# EUROPEAN PATENT APPLICATION

(11) **EP 1 932 537 A1**
(43) Date of publication of application: **18.06.2008**
(21) Application number: 06125897.6
(22) Date of filing: 12.12.2006
(51) Int. Cl.: A61K 39/00, C12N 5/06, A61P 35/00, A61P 37/00

(54) **Expression of transgenic T cell receptors in LAK-T cells**

(71) Applicant: Helmholtz Zentrum München Deutsches Forschungszentrum für Gesundheit und Umwelt (GmbH), 85764 Neuherberg (DE)
(72) Inventor: Schendel, Dolores, Jean, 80469, München (DE)
(74) Representative: Behnisch, Werner

(57) **Abstract**

The present invention is directed to LAK-T cells, which have been transformed by a transgenic T cell receptor (tg-TCR). The invention is further directed to a method of generating those transgenic T cells, a pharmaceutical composition comprising said cells and the use of the LAK-T cells or of the pharmaceutical composition in the adoptive cell therapy and for treating hematological malignancies or solid tumors or acute or chronic infections or autoimmune diseases.

## Description

The present invention is directed to LAK-T cells, which have been transformed by a transgenic T cell receptor (tg-TCR). The invention is further directed to a method of generating those transgenic T cells, a pharmaceutical composition comprising said cells and the use of the LAK-T cells or of the pharmaceutical composition in the adoptive cell therapy and for treating hematological malignancies or solid tumors or acute or chronic infections or autoimmune diseases.

### Background of the invention

The adoptive transfer of lymphocytes in the setting of allogeneic stem cell transplantation (SCT) has demonstrated the power of the immune system for eradicating hematological malignancies (Kolb et al. 1995). It appears that SCT can also function to eliminate solid tumors, such as renal cell carcinomas (RCC) in some cases (reviewed in Kolb et al. 2004 and Dudley and Rosenberg, 2003). In SCT recipients, the elimination of malignant cells may only occur after several months up to a year, due to the fact that specific T cells must be activated *in vivo* and must then expand to adequate numbers following the development of the new hematopoietic system in the transplant recipient. Alternatively, after a period of time (approximately 60 days) during which tolerance is established in the SCT recipient, a transfer of unprimed, unseparated leucocytes can be made to speed up the generation of immune responses directed against tumor cells. Here again, the specific lymphocytes capable of attacking tumor cells must be activated and expanded from the low frequency precursor lymphocytes that are present among the unselected population of leucocytes that are transferred. Donor leucocyte infusions (DLI) of unselected lymphocyte populations after SCT work well for the elimination of chronic myelogenous leukemia (CML), which grows slowly, but are less effective in the eradication of acute leukemia, partly due to the fact that the growth of the malignant cells outpaces the expansion capacity of the immune cells. This same expansion differential also impacts on the poor immune elimination of rapidly progressing solid tumors. A second handicap in the use of unselected leucocyte populations in DLI is that T cells may also be transferred that have the capacity to attack normal cells and tissues of the recipient, leading to graft-versus-host-disease (GVHD), a disease with high morbidity and mortality.

Recent studies have demonstrated that the adoptive transfer of selected T cells with defined tumor-associated specificities can lead to major reductions in tumor burden in an autologous setting, particularly if patients have been pretreated with non-myeloablative regimens (Dudley et al. 2002, 2003). This eliminates the need to perform SCT in a tumor patient, and thereby also bypasses the problem of GVHD. Effective immune responses were seen in pretreated melanoma patients who received mixtures of autologous tumor-infiltrating lymphocytes (TIL). These mixtures of cells, containing both CD4 and CD8 positive T cells, appear to be clinically more efficacious than the adoptive transfer of large numbers of a single CD8 T cell clone specific for a particular MHC-tumor-associated antigen (TAA) ligand. One factor contributing to this difference is the requirement to have CD4 T cells to maintain long-lived CD8 T cells *in vivo.* Furthermore, immune responses directed against single ligands can lead to the selection of tumor cell variants that have lost expression of the corresponding ligand and thereby can escape immune detection. On the one hand, transfer of complex mixtures of T cells, as they are present among tumor infiltrating lymphocytes, can overcome these problems by providing CD4 and CD8 cells with multiple specificities, but they can also lead to autoimmunity if the mixtures of TIL contain T cells that recognize ligands expressed by normal tissues. This is demonstrated, for example, by the attack of normal melanocytes leading to vitiligo in melanoma patients following adoptive cell therapy (ACT) using cells that recognize melanoma differentiation antigens that are also expressed in melanocytes (Dudley et al. 2002).

In order to extend the capacity to use ACT to treat patients with more rapidly growing tumors, it is a goal to transfer enriched, peptide-specific effector T cells (both CD4 T helper cells and cytotoxic T lymphocytes) that have been selected for their ligand specificities to effectively attack tumor cells while avoiding serious attack of normal tissues. These cells are to be rapidly expanded to large numbers *in vitro* and then used for ACT. Alternatively, the T cell receptors (TCRs) of such ligand-specific T cells can be cloned and expressed as TCR-transgenes in activated lymphocytes, using either recipient peripheral blood lymphocytes or activated T cell clones with defined specificities that grow well and do not have the capacity to attack normal host tissues.

US Patent Application 20020090362 discloses a method of treating a patient, the method comprising administering to the patient a therapeutically effective amount of cytotoxic T lymphocytes (CTL) which recognise at least part of an antigenic molecule when presented by an HLA class I (or equivalent) molecule on the surface of a cell wherein the cytotoxic T lymphocytes are not derived from the patient. This application describes the use of stimulating cells which are allogenic or even xenogenic in view of the donor of the CTL. US Patent Application 20020090362 further discloses stimulating cells which are preferably incapable of loading a selected molecule (peptide) and in particular the use of TAP deficient stimulating cells.

Examples of cells which show non-MHC-restricted immune response are Lymphokine Activated Killer (LAK) cells. They represent mixtures of activated natural killer (NK) and non-MHC-restricted T cells that lyse various tumor cells as well as HLA class I negative target cells. Activated NK cells, like those present in LAK populations, are inhibited in their cytolytic function following interaction with MHC class I molecules.

LAK cells represent a composite population of CD3⁻ NK cells and CD3⁺ T cells, of both the CD4 and CD8 subsets. Characteristic of LAK cells is their capacity to lyse a variety of tumor cells in a non-MHC-restricted fashion. In addition, LAK cells can kill class I negative target cells, such as Daudi and K562, which serve as general standards for identification of non-MHC-restricted cytotoxic effector cells. Separation of LAK populations into CD3⁻ and CD3⁺ fractions revealed that both NK cells and T cells could lyse class I negative target cells and a variety of different tumor cells. Furthermore the LAK-T cells can also be inhibited by expression of particular MHC class I molecules on target cells.

In practice, LAK cells show one major advantage over other non-MHC-restricted T-cells/NK cells, which is the relatively simple way in which these cells can be produced *in vitro.* LAK cells may be derived from peripheral blood mononuclear cells (PBMC) through *in vitro* culture in the presence of high dose interleukin-2 (IL-2). This method is superior to other ways of producing human NK cells as well as non-MHC-restricted CD4⁺ and CD 8⁺ T cells, e.g. by allogeneic stimulation of mixed peripheral blood lymphocyte (PBL) populations, since it involves only the use of cells from one donor and one cytokine.

EP 1 275 400 is directed to therapeutic compositions containing non-MHC-restricted T-cells/NK-cells in combination with MHC-restricted T-cells and especially to therapeutic compositions, which comprise LAK cells. The use of the above combination in the treatment of tumors in humans, which tumors show a missing, low or aberrant expression of MHC class 1a or 1b molecules is disclosed. By using the aforementioned compositions/combinations it is possible to provide a balanced selective pressure against emergence of tumor cell variants that would otherwise escape immune detection. An expression of transgenic TCR, however, is not disclosed in this patent.

The present approaches of using T cells expressing transgenic TCRs are suffering from the problem that the recipient cells may result in an attack of normal tissues of the patient, thus, leading to autoimmune diseases or GvHD. One solution to this problem is to use selected T cell lines or clones, where the exact specificity of the enodgenous TCRs is known. This, however, is time and cost intensive and may require the use of selection processes (such as tetramers or streptamers) that are not readily compliant with good manufacturing practice (GMP).

### Summary of the invention

Therefore, it is an object underlying the invention to provide T cells that bear tg-TCRs that have the capacity to recognize their MHC-peptide ligands on pathogenic agents, which T cells can be generated in large numbers and in a comparably short period of time and without a risk of causing autoimmune diseases or graft-versus host disease. It is a further problem underlying the present invention to provide a method for the rapid generation of antigen-specific T cells which can be used in adoptive cell transfer. Furthermore, it is a problem underlying the invention to provide a non-MHC-restricted T cell based pharmaceutical composition that can be used for treating a patient suffering from a disease without a risk of graft-versus-host-disease, or autoimmunity.

These problems are solved by the subject-matter of the independent claims. Preferred embodiments are set forth in the dependent claims.

The invention solves both problems by the use of lymphokine-activated T-cells (LAK-T cells).

First, the use of lymphokine stimulation, for example using cytokines like IL-2 or IL-15, can provide large numbers of such cells in short periods of time (1-2 weeks).

Second, LAK-T cells have the property of recognizing and efficiently killing target cells that have low or aberrant expression of MHC class I molecules, as is often the case with tumor cells. On the other hand, they are inhibited in their killing of normal body cells that express normal levels of MHC class I molecules.

Thus if LAK-T cells are transfected to express TCRs with tumor specificities they should be better activated to kill tumor cells that express the corresponding tg-TCR ligands, but they should not unduly attack normal cells which are protected by adequate expression of MHC molecules that turn-off the functions of LAK-T cells.

### Thereby, the disadvantages of the prior art approaches can be avoided which are:

Selection of T cell lines or clones with defined antigen specificities for use as tg-TCR recipients for each patient is:
■ Time consuming to isolate such cells
■ Technically not always feasible, so some patients may be denied therapy
■ Costly because of reagents and personal
■ Hard to comply with GMP standards or
■ Transfer of unselected, activated lymphocytes which are not negatively regulated by MHC class I molecules, which express a variety of endogenous TCRs, some of which cause autoimmunity or GvHD

Important for the present concept is that the recipient T cells for the TCR transgenes must kill target cells independent of MHC expression, and they should do this independent of their T cell receptors and most importantly, they should be negatively regulated by MHC class I molecules (either qualitatively or quantitatively). It is this latter property that will protect normal tissues against potential autoimmune attack.

LAK-T cells have the characteristic that they are negatively regulated by autologous MHC class I molecules and therefore it is possible to harness this natural mechanism to protect normal cells against unwanted attack by TCR-transgenic lymphocytes bearing a variety of different endogenous TCRs with unknown specificities. Recipient cells bearing tg-TCR can be activated through transgenic TCR signaling by appropriate tumor cells to overcome their negative regulation by MHC class I molecules, but will still retain their capacity to be negatively regulated through signaling to their inhibitory receptors by normal cells expressing self-MHC molecules, but which do not express the corresponding transgenic TCR-ligands. The use of such T cells in a clinical setting brings the potential advantage that they may also attack tumor cells showing aberrant expression of MHC class I allotypes that would allow escape from transgenic-TCR recognition.

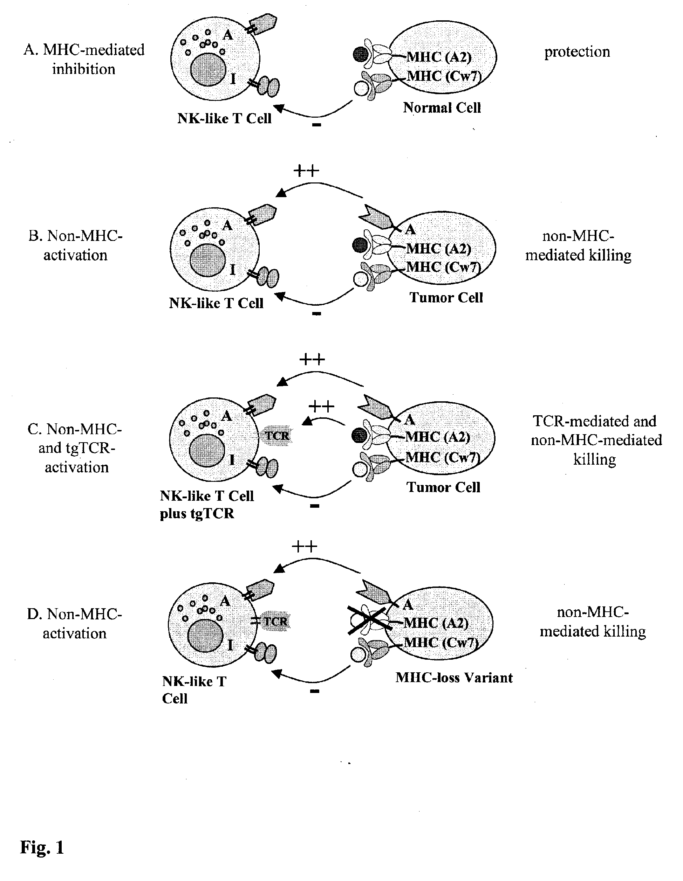

The combination of both approaches, using LAK-T cells, as recipients and providing them with tg-TCRs of desired specificity will provide the possibility to obtain high numbers of activated lymphocytes within a short period of time which use negative MHC regulation to guard against autoimmune damage of normal tissues (see Figure 1). The present approach thus is:
Fast, cheap, available for all patients, can be adapted to closed bag system. If tumor variants arise that no longer express the tg-TCR ligand they should still be susceptible to LAK-T-mediated killing that is not MHC-restricted.

Figure 1 illustrates the advantages of the invention:
LAK-T cells express activating receptors (AR), inhibitory receptors (IR) and endogenous TCRs (not shown). Normal cells are protected from killing because they fail to express activating ligands (AL) that stimulate AR but they do express MHC class I ligands that stimulate IR (see (A). In (B), tumor cells are killed by LAK-T cells in an endogenous TCR-independent manner when they receive signals to their AR that are greater than signals delivered to their IR by MHC class I molecules. Moreover, LAK-T cells that express transgenic TCRs (tg-TCRs) can kill tumor cells by the mechanism described in (B) and will be further activated through recognition of peptide-MHC ligands seen by their tg-TCRs (see (C). (D) If tumor cell variants lose expression of the peptide-MHC ligands of the tg-TCR, they should still remain susceptible to LAK-T killing through activation of their AR which is greater than inhibition by IR.

### Detailed description of the invention

According to a first aspect, the invention provides a LAK-T cell, which has been transformed by a transgenic T cell receptor (tg-TCR).

As mentioned above, several advantages can be derived from the use tg-TCR-transfomed LAK-T cells, being illustrated in Fig. 1 and in general above.

In a preferred embodiment, the LAK-T cell preferably is an activated Lymphokine Activated Killer (LAK) T cell of the CD4 or CD8 subtype.

The LAK-T cell of the invention preferably has been transformed by a vector encoding the tg-TCR. This vector is preferably an expression vector which contains a nucleic acid according to the invention and one or more regulatory nucleic acid sequences. Preferably, this vector is a plasmid or a retroviral vector.

Retroviral vectors are among the most preferred vectors. As an example for retroviral vectors to be used, one can name mouse myeloproliferative sarcoma virus (MPSV) based retroviral vectors, as well as, for example, the retroviral vector MP71-GFP-PRE (Engels et al. Hum. Gene Ther., 2003) and the vector particles are produced as described (Engels et al. Hum. Gene Ther., 2005; Sommermeyer et al. Eur. J. Immunol. 2006).

As an alternative, the tg-TCR is introduced into the activated T cell by transferring a nucleic acid coding for the TCR into said T cell by electroporation. For example, *in vitro* transcribed RNA can be prepared from cDNAs encoding TCR alpha and TCR beta chains and electroporated into the activated T cells, as described recently (Zhao et al. Mol. Ther., 2006).

The tg-TCR for transforming LAK-T cells are specific for a predefined MHC-antigen complex.

These antigens are preferably selected from pathogenic agents derived from viruses, bacteria, protozoa, and parasites as well as tumor cells or tumor cell associated antigens, autoantigens or functional parts thereof.

The viruses are preferably selected from the group consisting of influenza viruses, measles and respiratory syncytial viruses, dengue viruses, human immunodeficiency viruses, human hepatitis viruses, herpes viruses, or papilloma viruses. The protozoa may be Plasmodium falciparum, the bacteria tuberculosis-causing Mycobacteria.

The tumor associated antigen is preferably selected from hematological malignancies or solid tumors, more preferably colon carcinoma, breast carcinoma, prostate carcinoma, renal cell carcinoma (RCC), lung carcinoma, sarcomas or melanoma cells.

The non-MHC-restricted T cell of the invention preferably are derived from mixed lymphocyte populations from peripheral blood through *in vitro* culture in the presence of interleukin-2 (IL-2) and/or interleukin-15 (IL-15).

In a second aspect, the present invention provides a method of generating transgenic LAK-T cells comprising the following steps:
a) providing a mixed lymphocyte population;
b) activating those lymphocytes by suitable means;
c) enriching the activated cells and isolating LAK-T cells therefrom;
d) transforming the isolated LAK-T cells with a nucleic acid coding for a transgenic TCR specific for a predefined MHC-antigen complex.

In step a), lymphocyte populations preferably are derived from peripheral blood. See also chapter "Starting lymphocyte populations" in chapter Examples.

In particular, two sources of lymphocytes can be used for developing non-MHC-restricted effector cells. First, lymphocytes can be isolated from sterile peripheral blood samples, and, as an alternative, when larger numbers of lymphocytes are required, they can be obtained through the process of leukapheresis followed by elutriation, allowing 10-20 times more lymphocytes to be obtained. Both approaches are appropriate in order to practice step a) of the present method.

In step b), the activation can preferably be done by activating the lymphocytes of the invention preferably through *in vitro* culture in the presence of interleukin-2 (IL-2) and/or interleukin-15 (IL-15).

In step c), the activated cells are enriched and LAK-T cells are isolated from the mixture of lymphocytes. For example, an enrichment may be performed through depletion of other lymphocyte subsets (such as NK cells, B cells and regulatory T cells) using commercial reagents (Dynal Biotech, Oslo, Norway) according to manufacturer's instructions (Falk et al. Cancer Res. 2002). The enrichment process may be controlled by suitable means, for example by specific antibodies, and the enrichment may be repeated once or more if necessary.

As mentioned above, the nucleic acid is preferably comprised by a vector, more preferably, the vector is selected from a plasmid or a retroviral vector.

As an alternative, the tg-TCR may be introduced into the activated T cell by transferring a nucleic acid coding for the TCR into said T cell by electroporation, as mentioned above. For further information, it is also referred to chapter Example.

In a third aspect, a LAK-T cell is provided obtainable by the method as defined above.

In a still further aspect, a pharmaceutical composition comprising the above obtained LAK-T cells and a pharmaceutically acceptable carrier are provided.

The LAK-T cells of the present invention are preferably used in such a pharmaceutical composition in doses mixed with an acceptable carrier or carrier material, that the disease can be treated or at least alleviated. Such a composition can (in addition to the active component and the carrier) include filling material, salts, buffer, stabilizers, solubilizers and other materials, which are known state of the art.

The term "pharmaceutically acceptable" defines a non-toxic material, which does not interfere with effectiveness of the biological activity of the active component. The choice of the carrier is dependent on the application.

The pharmaceutical composition can contain additional components which enhance the activity of the active component or which supplement the treatment. Such additional components and/or factors can be part of the pharmaceutical composition to achieve synergistic effects or to minimize adverse or unwanted effects.

Techniques for the formulation or preparation and application/medication of active components of the present invention are published in "Remington's Pharmaceutical Sciences", Mack Publishing Co., Easton, PA, latest edition. An appropriate application is a parenteral application, for example intramuscular, subcutaneous, intramedular injections as well as intrathecal, direct intraventricular, intravenous, intranodal, intraperitoneal or intratumoral injections. The intravenous injection is the preferred treatment of a patient.

Preferably, the pharmaceutical composition of the invention is used for the manufacture of a medicament for adoptive cell therapy, in particular for treating hematological malignancies or solid tumors or acute or chronic infections or autoimmune diseases.

The present invention is illustrated by examples and the figure in the following.

Figure 1 is showing non-MHC-restricted T cells as recipient lymphocytes for transgenic TCRs. (A) Non-MHC-restricted T cells (LAK-T cells) express activating receptors (AR), inhibitory receptors (IR) and endogenous TCRs (not shown). Normal cells are protected from killing because they fail to express activating ligands (AL) that stimulate AR but they do express MHC class I ligands that stimulate IR. (B) Tumor cells are killed by LAK-T cells in an endogenous TCR-independent manner when they receive signals to their AR that are greater than signals delivered to their IR by MHC class I molecules. (C) LAK-T cells that express transgenic TCRs (tg-TCRs) can kill tumor cells by the mechanism described in B and will be further activated through recognition of peptide-MHC ligands seen by their tg-TCR. (D) If tumor cell variants lose expression of the peptide-MHC ligands of the tg-TCR, they should still remain susceptible to LAK-T killing through activation of their AR which is greater than inhibition by IR.

### Examples:

### Generation of non-MHC-restricted effector T cells

### Starting lymphocyte populations

Two sources of lymphocytes can be used for development of non-MHC-restricted effector cells. 1) Lymphocytes can be isolated from sterile peripheral blood samples using standard Ficoll gradient separation procedures. This process is suitable for obtaining up to 6 x 10e8 lymphocytes with blood samples of 300-500 mL or similarly, Ficoll separation of buffy coat cells can be used for isolation of lymphocytes. 2) When larger numbers of lymphocytes are required, they can be obtained through the process of leukapheresis followed by elutriation, allowing 10-20 times more lymphocytes to be obtained. Leukapheresis products are obtained, for example, through a 180 minute run using a modified mononuclear cell programme (Version 6.1), according to manufacturer's instructions, using a COBE Spectra (Gambro BCT, Lakewood, CO, USA) instrument. Thereafter, counter-flow centrifugal elutriation is performed with the ELUTRA instrument (Gambro, BCT) using a fixed rotor speed of 2400 rpm and computer-controlled stepwise adjustment of medium flow rate. The enriched lymphocytes, present in fraction 3, are washed twice in RPMI 1640 culture medium (Biochrom, Berlin, Germany) containing 1 % human serum albumin (Octalbine, Octapharma, Langen, Germany).

### Generation of LAK-T cells

The T cell fraction of lymphokine activated killer (LAK) cells provides one source of non-MHC-restricted effector T cells. These cells are generated by culturing the lymphocyte populations obtained by either of the two methods described above in RPMI 1640 culture medium , supplemented with 2 mM L-glutamine, 1 mM sodium pyruvate, 100 µM penicillin, 100 µg/mL streptomycin, containing 15% heat-inactivated pooled human serum and 500-1000 U/mL recombinant IL-2 (Proleukin, Cetus Corp., Emeryville CA.). ), with or without 1% phytohemagglutinin (PHA-P: Difco Laboratories, Detroit, MI). Cultures are incubated at 37° C in an atmosphere of 5% CO₂ for a period of time ranging normally from 3 to 7 days; however, the cells can be maintained in culture for longer periods of time. Thereafter, the activated T cell fraction is enriched through depletion of other lymphocyte subsets using commercial reagents (Dynal Biotech, Oslo, Norway) according to manufacturer's instructions (Falk et al. Cancer Res. 2002). Sample aliquots are controlled for enrichment by flow cytometry to detect binding of monoclonal antibodies specific for CD4 or CD8 surface molecules, according to the method described below. It is expected that greater than 95% of the cells are positive for CD4 or CD8 markers. Should this not be the case, then the enrichment procedure can be repeated once again.

### Generation on non-MHC-restricted T cells using enriched T cell subsets

### Enrichment of CD4 or CD8 lymphocyte fractions

Mixed lymphocyte populations obtained by either of the two methods described above are washed twice in phosphate buffered saline and then separated into CD4- or CD8-enriched fractions, using magnetic bead separation to deplete all other lymphocytes subsets using commercial reagents (Dynal Biotech, Oslo, Norway) according to manufacturer's instructions (von Geldern, Eur. J. Immunol, 2006). Sample aliquots are controlled for enrichment by flow cytometry to detect binding of monoclonal antibodies specific for CD4 or CD8 surface molecules, according to the method described below. It is expected that greater than 95% of the cells are positive for the CD4 or CD8 markers, respectively, dependent upon the specific population that is being enriched. Should this not be the case, then the enrichment procedure can be repeated once again.

### Stimulation of enriched lymphocytes

Enriched and washed lymphocytes are cultured in RPMI 1640 culture medium, supplemented with 2 mM L-glutamine, 1 mM sodium pyruvate, 100 µM penicillin, 100 µg/mL streptomycin, containing 15% heat-inactivated pooled human serum and 500-1000 U/mL recombinant IL-2 (Proleukin, Cetus Corp., Emeryville CA.). ) or 5 ng/mL IL-15 (PromoCell, Heidelberg, Germany), with or without 1% phytohemagglutinin (PHA-P: Difco Laboratories, Detroit, MI). Cultures are incubated at 37° C in an atmosphere of 5% CO₂ for a period of time ranging normally from 3 to 7 days; the cells can however be maintained in culture for longer periods of time. Sample aliquots are controlled by flow cytometry to detect binding of monoclonal antibodies specific for CD4 or CD8 surface molecules, and the activation marker CD45RO, according to the method described below (von Geldern, Eur. J. Immunol. 2006).

### Phenotyping of activated effector cells by flow cytometry

T cells are characterized for surface staining with the following monoclonal antibodies: FITC-conjugated anti-human CD3 (UCHT1), CD4 (13B8.2), CD8 (B9.11), and CD45RO (UCHL1), all purchased from Beckman-Coulter, Westbrook, ME. Lymphocytes are incubated for 45 min on ice with mAb, washed with phosphate buffered saline and 5% fetal bovine serum, fixed with PBS/1% paraformaldehyde and analyzed by flow cytometry (FACSCalibur; Becton Dickinson, San Jose, CA). Positive staining for the appropriate subset markers (CD4 or CD8) is expected on more than 95% of cells and presence of CD45RO, as a marker of activated/memory cells, is expected on more than 90% of cells (von Geldern, Eur. J. Immunol. 2006).

### Functional characterization

Two important functional characteristics are critical for assessing non-MHC-restricted effector T cells. As the name suggests, they should recognize target cells independent of expression of particular MHC molecules, which is a hallmark of recognition by classical MHC-restricted T cells that have a similar phenotype but which are only activated when they receive signals through their T cell receptors (TCRs). Non-MHC-restricted function can be assessed by measuring the capacity of the test populations to kill target cells that do not express MHC class I molecules, including Daudi, K562 and L721.221 cells (Falk et al. Cancer Res. 2002; von Geldern, Eur. J. Immunol. 2006). This function is prominent in the CD8 populations of non-MHC-restricted T cells and is less prevalent in CD4 cells, although some CD4 cells also show this capacity. Furthermore, when these MHC class I negative target cells are transfected to express particular MHC class I alleles, they become resistant to lysis by non-MHC-restricted effector T cells and this resistance can be reversed by blocking the target cells with antibody specific for MHC class I molecules. This reveals that the effector T cells are negatively regulated by MHC class I molecules (Falk et al. Cancer Res. 2002; von Geldern, Eur. J. Immunol. 2006).

Alternatively, non-MHC-restricted effector cells can be stimulated to secrete a variety of cytokines independent of TCR signalling upon contact with target cells. Cytokine secretion is prominent in the CD4 non-MHC-restricted effector T cells, but CD8 cells also can secrete cytokines. Of particular importance is the capacity of the CD4 T cells to secrete IFN-gamma.

### Cell-mediated cytotoxicity and blocking with monoclonal antibodies

Cell-mediated cytotoxicity is quantified in standard 4-h 51Cr-release assays (Schendel et al., Tissue Antigens, 1979). Target cells are labeled 90 min with Na₂⁵¹CrO₄, washed twice and 2x10e3 cells are exposed to effector cells for 4 h at varying effector to target cell ratios. Spontaneous isotope release is determined by incubating target cells alone, maximal release is determined by directly counting labelled target cells. Duplicate or triplicate measurements of four-step titrations of effector cells are generally used. For MHC blocking, target cells expressing defined class I alleles (such as L721.112; Falk et al. Cancer Res. 2002; von Geldern, Eur. J. Immunol. 2006) are incubated 30 min at 37° C with HLA-specific mAb A1.4 (Olympus, Hamburg, Germany) or isotype mAb (MOPC21; 10 1g/mL) and then combined with effector cells in a chromium-release assay. Changes in chromium release of target cells incubated with A1.4 antibody is compared to isotype control antibody. Higher levels of chromium release are expected in the presence of A1.4 antibody as compared to isotype control, indicating that masking of MHC class I on the target cell reverses the resistence caused by MHC class I molecules interacting with inhibitory receptors on the non-MHC-restricted effector cells (Falk et al. Cancer Res. 2002; von Geldern, Eur. J. Immunol. 2006).

### Cytokine and chemokine secretion

Cytokines/chemokines are quantified by multiplex protein arrays according to manufacturer's instructions (Bio-Rad Laboratories, Hercules, CA, USA). Microspheres coated with cytokine-specific mAb are incubated for 30 min at room temperature, with 50 µL supernatant. After three washing steps, biotinylated detection mAb are added, incubated for 30 min at room temperature, followed by streptavidin-PE incubation. A two-laser array reader (Luminex v-Map) simultaneously quantifies 11 cytokines; standard curves (3.91-3.2x10e4 pg/mL) and concentrations are calculated with Bio-Plex Manager 3.1. Cytokines and chemokines that can be assessed with this method include: IL-2, IL-4, IL-5, IL-6, IL-8, IL-10, IL-13, GM-CSF, IFN-gamma, TNF-alpha, MIP-1beta.

### Introduction of transgenic TCR sequences into non-MHC-restricted T cells

### Construction of retroviral vectors and production of virus supernatants

The TCRα and TCRβ chain genes of a TCR of selected specificity are amplified by PCR and cloned into the retroviral vector MP71-GFP-PRE (Engels et al. Hum. Gene Ther., 2003) and vector particles are produced as described (Engels et al. Hum. Gene Ther., 2005; Sommermeyer et al. Eur. J. Immunol. 2006).

### Transduction of T cells

T cells are transduced in 24-well non-tissue culture plates coated with RetroNectin (3.5 µg/well) (Takara, Apen, Germany). Activated human T cells are incubated with retrovirus supernatant, supplemented with protamine sulfate (final concentration 4 µg/ml) (Sigma-Aldrich, Munich, Germany). After addition of supernatant, the plates are spinoculated with 800 g for 1.5 h at 32°C. Medium was replaced by fresh culture medium after 48-72 h (Engels et al. Hum. Gene Ther., 2005; Sommermeyer et al. Eur. J. Immunol. 2006).

### Electroporation of in vitro transcribed RNA

Alternatively, *in vitro* transcribed RNA can be prepared from cDNAs encoding TCR alpha and TCR beta chains and electroporated into the activated T cells, as described recently (Zhao et al. Mol. Ther., 2006).

### Phenotyping of TCR-transgenic non-MHC-restricted effector cells

Expression of the transgenic T cell receptor (TCR) in non-MHC-restricted effector cells can be determined with flow cytometry by one of two methods. A panel of monoclonal antibodies detecting the different human V-beta chains is commercially available (Immunotech, Marseille, France). Based on the known sequence of the transgenic V-beta chain, the transduced cells can be stained using the corresponding antibody. Alternatively, if fluorescence-labeled MHC multimers, representing the MHC-peptide complex seen by the selected TCR, are available they can be used in flow cytometry to detect transgenic TCR expression on the non-MHC-restricted effector cells. Fluorescence intensity of labelled bound antibody or multimer is measured using a FACSCalibur flow cytometer and Cellquest Pro software (Becton Dickinson, Heidelberg, Germany).

### Functional analysis of tg-non-MHC-restricted effector cells

The effector cells that now express transgenic TCRs have different functional capacities from the original non-MHC-restricted effector cells. The transgenic effector cells retain their capacity to recognize and kill MHC class I negative target cells, as described above. This function is not disturbed by blocking with antibodies intereacting with TCRs since recognition occurs independently of the TCR (Falk et al., J. Exp. Med., 1995). This non-MHC-restricted effector cell function can be ascertained by the methods described above (cytotoxicity assays and blocking with MHC antibodies). Following introduction of the transgenic TCR into the non-MHC-restricted effector cells they now gain an additional MHC-restricted TCR specificity. This new specificity can be ascertained by testing their capacity to recognize and kill target cells expressing the MHC-peptide complex that represents the ligand for the transgenic TCR. In contrast to the non-MHC-restricted effector cell function, this recognition is dependent upon TCR signalling and therefore can be blocked by antibodies specific for CD3, or if a suitable antibody for the V-beta chain is available also by antibody blocking with such a TCR-specific reagent. In addition, this recognition is also inhibited by blocking MHC class I expression on the target cells. Here cytotoxicity is expected to be decreased in the presence of class I-specific antibody as compared to isotype control antibodies. Thus, by testing different target cells, which do or do not express MHC class I molecules and do or do not express the MHC-peptide ligand of the transgenic TCR, combined with blocking studies using either antibodies specific for the transgenic TCR, CD3 on the effector cell side and antibodies specific for MHC molecules on the target cell side, it is possible to demonstrate that both effector cell functions are present in the TCR-transgene expressing, non-MHC-restricted effector T cell population.

A particular advantage of this approach is that both specificities, MHC-restricted recognition of tumor cells via the transgenic TCR and the inherent tumor cell recognition mediated by the non-MHC-restricted effector cells can both be harnessed for tumor attack with the same population of effector cells. Furthermore, this means that it is not necessary to obtain extremely high percentages of transgenic TCR-expressing lymphocytes, thereby avoiding the need to enrich transduced or electroporated recipient cells for transgene expression, since benefit through the non-MHC-restricted cytotoxicity and/or cytokine secretion is expected from the non-MHC-restricted effector cells that do not express the transgenic TCR. The use of both CD4 and CD8 populations of non-MHC-restricted effector cells provides complementary functions of high anti-tumor cell cytotoxicity and cytokine secretion.

### References

Engels, B, Cam, H., Schuler, T., Indraccolo, S., Gladow, M., Braun, C., Blankenstein, T., and W. Uckert. 2003. Retroviral vectors for high-level transgene expression in T lymphocytes. Hum. Gene Ther. 14: 1155-1168.
Engels, B., Nössner, E., Frankenberger, B., Blankenstein, Th., Schendel, D.J., and W. Uckert. 2005. Redirecting human T lymphocytes towards renal cell carcinoma-specificity by retroviral transfer of T cell receptor genes. Human Gene Ther. 16(7):799-810.
Falk, C.S. Noessner, E., Weiss, E.H. and D.J. Schendel. 2002. Retaliation against tumor cells showing aberrant HLA expression using lymphokine activated killer (LAK)-derived T cells. Cancer Res. 62 (2): 480-487.
Falk, C.S., Steinle, A. and D.J. Schendel. 1995. Expression of HLA-C molecules confers target cell resistance to some non-MHC-restricted T cells in a manner analogous to allospecific NK cells. J. Exp. Med. 182:1005-1018.
Schendel, D.J., Wank, R. and B. Dupont. 1979. Standardization of the human in vitro cell-mediated lympholysis technique. Tissue Antigens 13: 112-120.
Sommermeyer, D., Neudorfer, J., Weinhold, M., Leisegang, M., Engels, B., Noessner, E., Heemskerk, M.H.M., Schendel, D.J., Blankenstein, T., Bernhard, H., and W. Uckert. 2006. Designer T cells by T cell receptor replacement. Eur. J. Immunol. 36: 3052-3059.
von Geldern, M., Simm, B., Braun, M., Weiss, E.H., Schendel, D.J. and Falk, C.S. 2006. TCR-independent cytokine stimulation induces non-MHC-restricted cell activity and is negatively regulated by HLA class I. Eur. J. Immunol. 36: 2347-2358. [Epub Aug 15, 2006].
Zhao, Y., Zheng, Z., Cohen, C., Gattiononi, L., Palmer, D.C., Restifo, N.P., Rosenberg, S.A. and R.A. Morgan. 2006. High-efficiency transfction of primary human and mouse T lymphocytes using RNA electroporation. Mol. Ther. 13(1): 151-159.

## Claims

1. An activated Lymphokine Activated Killer (LAK) T cell, which has been transformed by a transgenic T cell receptor (tg-TCR).

2. The LAK-T cell of claim 1, which is of the CD4 or CD8 subtype.

3. The LAK-T cell of claim 1, wherein the cell has been transformed by a vector encoding the tg-TCR.

4. The LAK-T cell of claim 3, wherein the vector is selected from the group consisting of a plasmid and a retroviral vector.

5. The LAK-T cell of claim 3, wherein the tg-TCR is introduced into the activated T cell by transferring a nucleic acid coding for the TCR into said T cell by electroporation.

6. The LAK-T cell of claim 5, wherein the nucleic acid coding for the TCR is RNA prepared from cDNA encoding TCR alpha and beta chains.

7. The LAK-T cell of one or more of the preceding claims, wherein the tg-TCR is specific for a predefined MHC-antigen complex.

8. The LAK-T cell of claim 7, wherein the antigen is selected from pathogenic agents derived from viruses, bacteria, protozoa, and parasites as well as tumor cells or tumor cell associated antigens, autoantigens or functional parts thereof.

9. The LAK-T cell of claim 8, wherein the viruses are selected from the group consisting of influenza viruses, measles and respiratory syncytial viruses, dengue viruses, human immunodeficiency viruses, human hepatitis viruses, herpes viruses, or papilloma viruses or wherein the protozoa is Plasmodium falciparum or the bacteria is tuberculosis-causing Mycobacteria.

10. The LAK-T cell of claim 8, wherein the tumor associated antigen is selected from hematological malignancies or solid tumors, preferably colon carcinoma, breast carcinoma, prostate carcinoma, renal cell carcinoma (RCC), lung carcinoma, sarcoma or melanoma cells.

11. The LAK-T cell of one or more of the preceding claims, wherein the cells are derived from peripheral blood mononuclear cells (PBMC) through *in vitro* culture in the presence of interleukin-2 (IL-2) and/or interleukin-15 (IL-15).

12. A method of generating transgenic LAK-T cells comprising the following steps:
a) providing a mixed lymphocyte population from peripheral blood;
b) activating those lymphocytes by suitable means;
c) enriching the activated cells and isolating LAK-T cells therefrom;
d) transforming the isolated LAK-T cells with a nucleic acid coding for a transgenic TCR specific for a predefined MHC-antigen complex.

13. The method of claim 12, wherein the LAK-T cell is an activated Lymphokine Activated Killer (LAK) T cell, preferably of the CD4 or a CD8 subtype.

14. The method of claim 12 or 13, wherein the lymphocytes are activated through *in vitro* culture in the presence of interleukin-2 (IL-2) and/or interleukin-15 (IL-15).

15. The method of claim 12 - 14, wherein the nucleic acid is comprised by a vector.

16. The method of claim 15, wherein the vector is selected from a plasmid or a retroviral vector.

17. The method of claim 12 - 14, wherein the tg-TCR is introduced into the activated T cell by transferring a nucleic acid coding for the TCR into said T cell by electroporation.

18. The method of claim 17, wherein the nucleic acid coding for the TCR is RNA prepared from cDNA encoding TCR alpha and beta chains.

19. A LAK-T cell obtainable by the method of one or more of claims 12-18.

20. A pharmaceutical composition comprising LAK-T cell of one or more of the preceding claims and a pharmaceutically acceptable carrier.

21. Use of the LAK-T cell of one or more of claims 1-11, 19 or of the pharmaceutical composition of claim 20 for the manufacture of a medicament for adoptive cell therapy.

22. The use of claim 21 for treating hematological malignancies or solid tumors or acute or chronic infections or autoimmune diseases.
